Europäisches Patentamt

European Patent Office

Office européen des brevets

(11) Veröffentlichungsnummer: **0 274 705 B1**

# EUROPÄISCHE PATENTSCHRIFT

(45) Veröffentlichungstag der Patentschrift: **04.03.92**

(51) Int. Cl.⁵: **A61M 23/00**, A61B 17/32

(21) Anmeldenummer: **87118774.6**

(22) Anmeldetag: **17.12.87**

(54) **Sonde zur Einführung in den menschlichen oder tierischen Körper, insbesondere Papillotom.**

(30) Priorität: **18.12.86 DE 3643362**

(43) Veröffentlichungstag der Anmeldung:
**20.07.88 Patentblatt 88/29**

(45) Bekanntmachung des Hinweises auf die
Patenterteilung:
**04.03.92 Patentblatt 92/10**

(84) Benannte Vertragsstaaten:
**AT BE CH DE ES FR GB GR IT LI LU NL SE**

(56) Entgegenhaltungen:
**EP-A- 0 019 930**
**DE-C- 3 347 122**
**GB-A- 1 208 639**
**US-A- 3 119 392**
**US-A- 4 080 706**

(73) Patentinhaber: **Frimberger, Erintrud**
**Josef-Kösel-Weg 10**
**W-8960 Kempten(DE)**

(72) Erfinder: **Frimberger, Eckart, Dr.**
**Tristanstrasse 12**
**W-8000 München 40(DE)**

(74) Vertreter: **Körber, Wolfhart, Dr.rer.nat. et al**
**Patentanwälte Dipl.-Ing. H. Mitscherlich**
**Dipl.-Ing. K. Gunschmann Dr.rer.nat. W. Kör-**
**ber Dipl.Ing. J. Schmidt-Evers Dipl.-Ing. W.**
**Melzer Steinsdorfstrasse 10**
**W-8000 München 22(DE)**

**Beschreibung**

Die Erfindung bezieht sich auf eine Sonde nach dem Oberbegriff des Anspruchs 1.

Die komplikationsärmste Methode zur Entfernung von Steinen aus dem Gallengang besteht heute in der endoskopisch durchgeführten Papillenspaltung mit nachfolgender Steinextraktion.

Hierzu wird mit einem peroral einzuführenden Seitblick-Duodenoskop nach Durchführung durch die Speiseröhre und den Magen der abführende Ast des Zwölffingerdarms (Duodenum) aufgesucht, wo der Gallengang und der Gang der Bauchspeicheldrüse in einer warzenförmigen Erhebung (Papille) gemeinsam münden.

Zunächst wird die Öffnung der Papille mit einer seitlich aus dem Duodenoskop ausschiebbaren Sonde intubiert. Mittels eines mit der Sonde eingespritzen Kontrastmittels werden die beiden Gänge röntgenologisch sichtbar gemacht, wobei sich Steine als Kontrastmittelaussparungen abzeichnen. Nach dieser Darstellung des Steines wird die Sonde entfernt, und statt ihrer wird - ebenfalls durch das Duodenoskop - ein sog. Papillotom (DE-C-3 347 122 und EP-A-0 019 930) in den Gallengang eingeführt und zwar soweit, daß der mittels des Zugdrahtes krümmbare Abschnitt des Papillotoms sich im Bereich der Papille befindet. Durch eine Zugausübung auf den Zugdraht an dessen äußerem Ende, bei der sich das Papillotom krümmt und der Zugdraht sehnenförmig spannt, und durch gleichzeitigen Anschluß des Zugdrahtes an eine Stromquelle wird die Papille in Übereinstimmung mit dem sich aufwärts erstreckenden Gallengang aufgeschnitten. Durch die erweiterte Papille kann dann der Stein spontan abgehen, oder er wird mit einem Werkzeug (Fangkorb) herausgezogen.

Aufgrund physikalischer Bedingungen weist die vom Zugdraht gebildete Bogensehne nach der Zugausübung immer nach oben, d.h. etwa in Richtung des Verlaufs des Duodenoskops. Hierdurch ist die Schnittrichtung etwa vorbestimmt, und es kann eine wahlweise seitliche Schnittrichtung praktisch nicht beeinflußt werden, ohne die Stellung des Duodenoskops zu verändern, was eine neue Orientierung des Duodenoskops erfordern würde. Dieser Sachverhalt ist insofern von erheblicher Bedeutung, da die Papille immer entsprechend des Verlaufs des sich nach oben erstreckenden Gallengangs bzw. des sich an die Papille anschließenden Wulstes geschnitten werden muß. Ein Schnitt seitlich über diesen Wulst hinaus bedeutet eine ernste Komplikation aufgrund einer Perforation mit eventuell tödlichem Ausgang.

Sowohl das Einführen der eingangs beschriebenen Sonde als auch das Aufschneiden der Papille mittels des Papillotoms entsprechend des aufsteigenden Verlaufs des Gallenganges ist bei der bekannten Ausgestaltung schwierig, weil die Krümmungsrichtung der Sonde bzw. des Papillotoms sich lediglich aufgrund der Zugspannung ergibt, jedoch nicht definiert ist, so daß sich wesentliche Abweichungen ergeben können.

Der Erfindung liegt die Aufgabe zugrunde, eine Sonde der eingangs bezeichneten Art so auszugestalten, daß eine definierte Krümmungsrichtung erreicht wird.

Diese Aufgabe wird durch die kennzeichnenden Merkmale des Anspruchs 1 gelöst.

Bei der erfindungsgemäßen Ausgestaltung ist die Krümmungsrichtung durch die Richtung, in der das geringere Widerstandsmoment des Mantels wirksam ist, vorbestimmt und somit stabilisiert. Dies trifft sowohl für den Fall zu, in dem das geringere Widerstandsmoment in die gleiche Richtung wirkt, in der auch die Zugspannung wirkt, als auch in dem Fall, in dem die Richtung des geringeren Widerstandsmomentes von der Richtung der Zugspannung abweicht. Im letzteren Fall läßt sich durch Vorgabe einer bestimmten Richtung des geringeren Widerstandsmomentes eine wahlweise Krümmungsrichtung erreichen, wodurch zusätzlich zu der angestrebten Stabilisierung eine Anpassung an die anatomischen Verhältnisse möglich ist.

Die unterschiedlichen Widerstandsmomente hinsichtlich rechtwinklig zueinander gerichteter Querschnittsrichtungen des Mantels lassen sich sowohl durch eine längliche Querschnittsform des Mantels als auch durch dem Mantel zugeordnete Zusatzelemente erreichen, die den vorgenannten entsprechende Widerstandsmomente aufweisen. Hierzu eignet sich insbesondere ein Biegeelement in Form einer Blattfeder, deren Biegungs- bzw. Krümmungsrichtung aufgrund des unterschiedlichen Breiten- und Dickenverhältnisses vorgegeben ist. Ein vorgenanntes Zusatzelement läßt sich in einfacher Weise dem Mantel zuordnen, z.B. durch Aufnahme in den vorhandenen Hohlraum des Mantels, durch Einbettung in die Wand des Mantels oder auch durch eine Anordnung an der Außenseite des Mantels.

Es ist auch von wesentlichem Vorteil, dem behandelten Arzt ein Sortiment erfindungsgemäßer Sonden zu Verfügung zu stellen, bei denen die durch die erfindungsgemäße Ausgestaltung vorgegebene Krümmungs- bzw. Biegerichtung von Sonde zu Sonde unterschiedlich ist, etwa in einer Stufung von 10 bis 15°. In diesem Fall vermag der behandelnde Arzt nach Kenntnis der anatomischen Verhältnisse einen passende Sonde auszuwählen. Hierdurch läßt sich auf einfache Weise eine bestimmte Biege- bzw. Krümmungsrichtung oder Schnittrichtung vorgeben und einhalten.

Es ist im Rahmen der Erfindung auch möglich, den Querschnitt des Mantels oder das Biegeelement in Anpassung an die anatomischen Verhältnisse oder die beabsichtigtigte Biegung bzw. Krümmung zu tordieren, wodurch sich ebenfalls eine Anpassung an vorgegebene bzw. beabsichtigte Verhältnisse erreichen läßt.

Nachfolgend wird die Erfindung anhand von in vereinfachten Zeichnungen dargestellten Ausführungsbeispielen näher erläutert. Es zeigt

Fig. 1 eine erfindungsgemäß ausgestaltete Sonde in Form eines Papillotoms in der Seitenansicht und teilweise im Schnitt;

Fig. 2 das innere Ende des Papillotoms im gekrümmten Zustand;

Fig. 3 bis 6 der Fig. 3 entsprechende Schnitte anderer Ausgestaltungen;

Fig. 7 ein Biegeelement für das Papillotom in der Seitenansicht;

Fig. 8 das Biegeelement in der Vorderansicht;

Fig. 9 einen Schnitt durch einen Zwölffingerdarm mit einer Funktionsdarstellung des Papillotoms;

Fig. 10 eine Ansicht auf die Papille des Zwölffingerdarms.

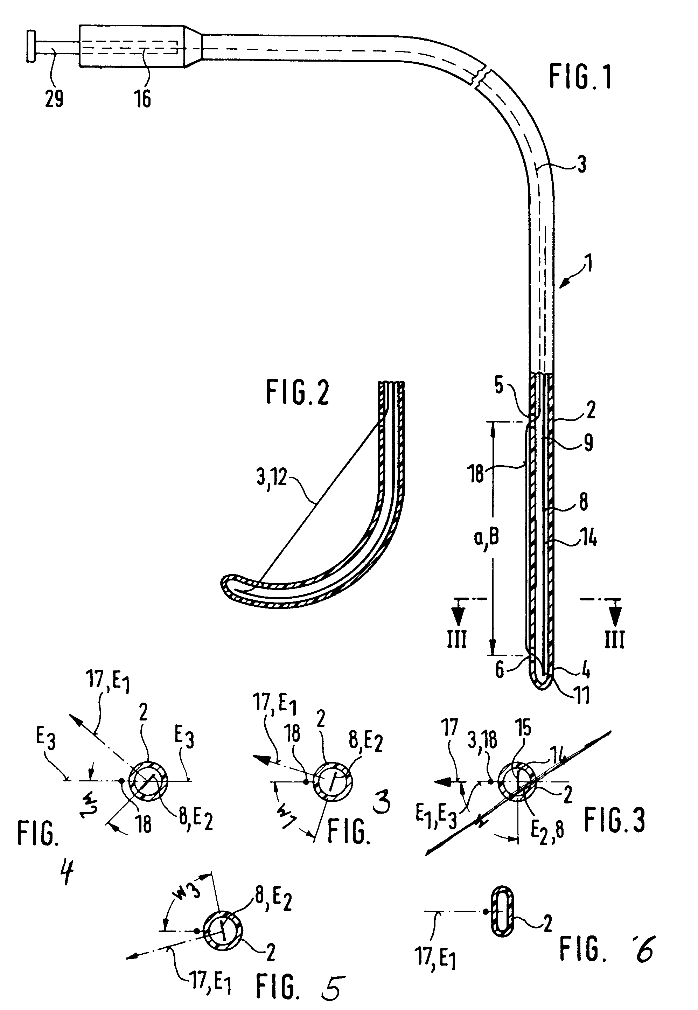

Das in Fig. 1 allgemein mit 1 bezeichnete Papillotom besteht aus einem Mantel 2, in dem ein Zugelement vorzugsweise in Form eines Drahtes 3 verläuft, der im Bereich des Einführungsendes des Papillotoms 1, d.h. im Bereich des inneren Endes des Mantels 2 an letzterem befestigt ist. Der Mantel 2 besteht aus einem biegeelastischen Rohr, vorzugsweise aus Kunststoff. Im Bereich des mit 4 bezeichneten inneren Endes weist der Mantel 2 auf einer axialen Linie in einem Abstand a voneinander zwei Löcher 5, 6 auf, von denen das innere, d.h. das dem inneren Ende 4 am nächsten gelegene Loch 6 nur einen geringen Abstand vom inneren Ende 4 aufweist. Der mit a bezeichnete Abstand beträgt etwa 2,5 cm.

Der Draht 3 ist durch das äußere Loch 5 herausgeführt, so daß er an der Außenseite des Mantels 2 verläuft, und in das innere Loch 6 wieder eingeführt. Die Befestigung des Drahtes 3 am Mantel 2 erfolgt durch die Befestigung des Drahtes 3 an einem Biegeelement in Form einer Blattfeder 8, die im Hohlraum 9 des Mantels 2 angeordnet ist, und an deren innerem Ende 11 der Draht 3 befestigt ist. Die Länge L der Blattfeder 8 ist größer bemessen, als die Länge des mit B bezeichneten Biegeabschnitts, im Bereich dessen bei einer Zugausübung auf den Draht 3 an dessen äußeren Ende das innere Ende 4 des Mantels 2 die in Fig. 2 dargestellte Krümmung einnimmt, bei der der Draht 3 eine Sehne 12 zum vorhandenen Krümmungsbogen darstellt. Mit der Sehne 12 lassen sich in bekannter Weise nach Anschluß eines elektrischen Stroms Schnitte im Gewebe des menschlichen oder tierischen Körpers ausführen.

In Fig. 1 ist die Blattfeder 8 mit ihrer Schmalseite 14 sichtbar, d.h. sie ist mit einer ihrer Breitseiten 15 dem bezüglich des Mantels 2 einseitig verlaufenden Drahtabschnitt 18 zugewandt. Bei einer Zugausübung auf den Draht 3 an dessen äußerem Ende 16 biegt die Blattfeder 8 in der mit $E_1$ bezeichneten Biegeebene ein, wie es in Fig. 2 dargestellt ist. Diese Biegeebene $E_1$ ist rechtwinklig zur Ebene $E_2$ der Blattfeder 8 gerichtet.

Aufgrund ihrer schmalen Querschnittsform ist das Widerstandsmoment gegen Biegung der Blattfeder 8 in der Biegeebene $E_1$ verhältnismäßig gering, während ihr Widerstandsmoment in ihrer eigenen Ebene $E_2$ verhältnismäßig groß ist. Die Blattfeder 8 übt somit eine Führung für die Biegebewegung aus, wodurch die Biegebewegung beträchtlich stabilisiert wird. Dies trifft auch für die Zurückbiegung des gekrümmten Abschnitts des Mantels 2 zu. Aufgrund des Vorhandenseins der Blattfeder 8 ist ein elastisches Material für den Mantel 2 nicht erforderlich, weil dieser durch die Blattfeder 8 zurückgebogen wird.

Es ist jedoch möglich und auch aus noch zu beschreibenden Gründen vorteilhaft, die Blattfeder 8 gemäß Fig. 3 bis 6 schräg zu der Ebene $E_3$ anzuordnen, die den außerhalb des Mantels 2 verlaufenden Drahtabschnitt 18 und die Mittelachse des Mantels 2 schneidet, wobei der sich jeweils ergebende spitze Winkel $w_1$, $w_2$, $w_3$ zwischen der Ebene $E_3$ und der Biegeebene $E_1$ der Blattfeder 8 rechts oder links vom Drahtabschnitt 18 liegen kann. Es empfiehlt sich, dem behandelnden Arzt ein mehrere Papillotome 1 umfassendes Sortiment zur Verfügung zu stellen, mit wenigstens einem Papillotom gemäß Fig. 1 und wenigstens ein oder zwei Papillotome 1, bei denen die Blattfeder 8 zu wenigstens einer Seite des Drahtabschnitts 18 geneigt schräg steht (Fig. 3 bis 6). Dabei ist es weiter vorteilhaft, Papillotome 1 mit Winkeln $w_1$, $w_2$ unterschiedlicher Größe dem Sortiment zuzuordnen, damit der behandelnde Arzt die von ihm gewünschte Schnittrichtung die im wesentlichen der Biegerichtung 17 der Blattfeder 8 entspricht, bestimmen kann. Der Winkelunterschied von Stufe zu Stufe beträgt vorzugsweise 10 bis 15°.

Die Abwandlung gemäß Fig. 6 zeigt einen rohrförmigen Mantel 2, der nicht durch ein zusätzliches Biegelement sondern aufgrund seiner Querschnittsform, einer länglichen, flach gedrückten Querschnittsform, hinsichtlich seiner Biegerichtung 17 stabilisert ist. Die Biegerichtung 17 ist bei diesem Beispiel durch die Richtung bestimmt, in die der Biegeabschnitt B des Mantels 2 sein aufgrund der länglichen Querschnittsform vorgegebenes geringeres Widerstandsmoment aufweist. Hier stimmt die Biegerichtung 17 mit der Ebene $E_1$ überein, d.h. der Biegeabschnitt B ist in der Ebene $E_1$, die auch Zugebene ist, stabilisert.

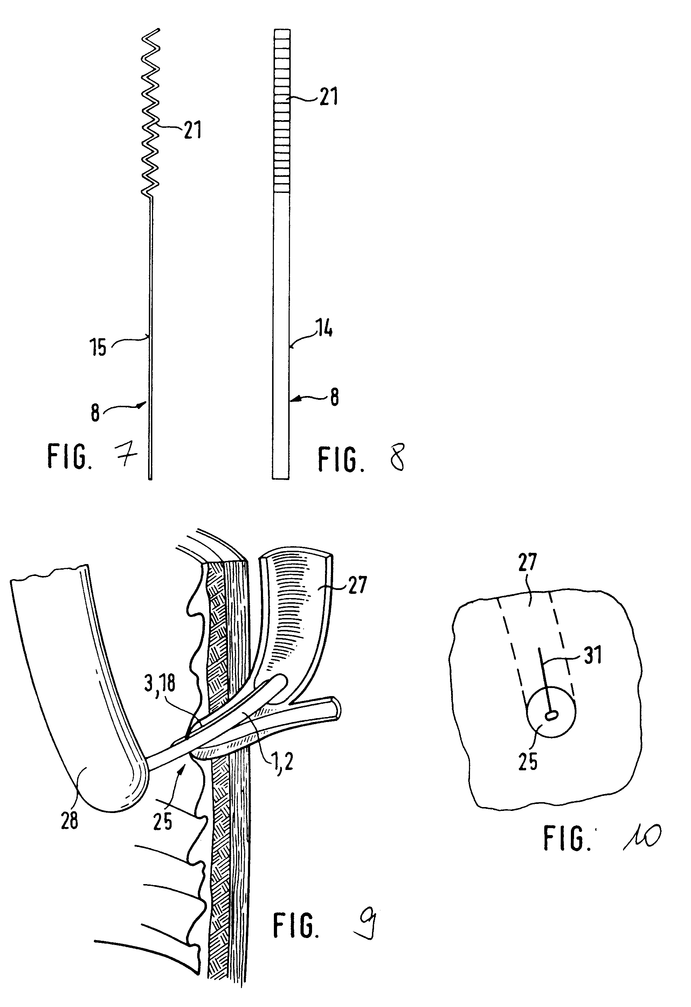

Gemäß den Fig. 7 und 8 erstreckt sich die Blattfeder 8 mit einem Verlängerungsabschnitt 21 über das äußere Loch 5 im Mantel 2 hinaus und zwar zwecks Stabilisierung des Mantels 2 in diesem Bereich. Der Verlängerungsabschnitt 21 ist zickzack- bzw. wellenförmig gebogen, und zwar so, daß der vom Verlängerungsabschnitt 21 benötigte Querschnittsraum dem Querschnitt des Hohlraums 9 angepaßt ist. Dies trägt sowohl zur Stabilisierung des Mantels 2 in diesem Bereich als auch zu einer stabilisierten Aufnahme der Blattfeder 8 im Mantel 2 bei und behindert den seitlichen Austritt aus einem noch zu beschreibenden Duodenoskop nicht.

Das Papillotom 1 dient dazu, die in Fig. 9 mit 25 bezeichnete Papille durch einen aufwärts gerichteten Schnitt zu weiten, um im Gallengang 27 befindliche, nicht dargestellte Steine entfernen zu können. Das Papillotom 1 wird mittels einem Seitblick-Duodenoskop 28 in die Papille 25 bzw. in den Gallengang 27 eingeführt, wie eingangs beschrieben. Dann wird durch die Zugausübung am in Fig. 1 mit 29 bezeichneten, am Draht 3 befestigten Zugglied der Draht 3 gespannt, wobei sich der Biegeabschnitt B krümmt (Fig. 2) und bei gleichzeitiger Verbindung des Drahtes 3 mit einer Stromquelle der in Fig. 11 mit 31 bezeichnete Schnitt ausgeführt wird.

In Fig. 10 ist deutlich erkennbar, daß der Schnitt 31 aufwärts und zwar längs des Gallengangs 27 erfolgen muß, da es andernfalls zu einer Perforation des Gallengangs mit schwerwiegenden Folgen kommt.

Mit der erfindungsgemäßen Ausgestaltung läßt sich die Schnittrichtung, die sich aufgrund der Zugausübung auf den Biegeabschnitt B automatisch etwa in Längsrichtung des Duodenoskops 28 einstellt, durch die Blattfeder 8 oder das besondere Querschnittsverhältnis des Mantels 2 sowohl stabilisieren als auch korrigieren.

Aufgrund unterschiedlicher anatomischer Verhältnisse ist es in vielen Fällen erforderlich, einen schrägen Schnitt 31 auszuführen, wobei die Schrägstellung von der Erstreckung des Duodenoskops 28 abweicht. In solchen Fällen ist es vorteilhaft, Papillotome 1 mit unterschiedlichen Winkeln $w_1$ bis $w_3$ zur Verfügung zu haben, um einen an die anatomischen Verhältnisse angepaßten Schnitt 31 ausführen zu können. Bei der praktischen Ausführung wird so vorgegangen, daß mit einem herkömmlichen Papillotom oder einem Papillotom 1 gemäß Fig 6 der Schnitt begonnen wird. Erweist sich die sich ergebende Schnittrichtung als nicht optimal, was in bekannter Weise durch das Duodenoskop 28 oder röntgenologisch beobachtet werden kann, wird das Papillotom ausgetauscht gegen ein Papillotom mit entsprechendem Schnittwinkel $W_1$ bis $W_3$. Die Ausrichtung der beabsichtigten Schnittbewegung kann somit erfolgen, ohne daß das Duodenoskop 28 verlagert werden muß.

Im Rahmen der Erfindung ist es möglich, andere Querschnittsformen des Mantels 2 und auch andere Biegeelemente oder andere Anordnungen eines Biegeelements vorzusehen. Es ist z.B. möglich, den Mantel zumindest in seinem Biegebereich B aus vollem Material zu bilden. Anstelle einer in den Hohlraum des Mantels 2 eingesetzten Blattfeder kann auch eine in das Material des Mantels eingebettete oder an den Mantel angeklebte Blattfeder benutzt werden. Es ist auch möglich, den Biegebereich B allein durch eine Blattfeder oder ein entsprechendes Biegeelement zu bilden.

**Patentansprüche**

1. Sonde (1) zur Einführung in den menschlichen oder tierischen Körper, mit einem Mantel (2) aus biegsamen Material, und mit einem im Mantel längs verlaufenden Zugelement (3), das zwecks Herbeiführung einer Krümmung durch Zugausübung an seinem äußeren Ende am Mantel (2) befestigt ist und im Krümmungsabschnitt (B) des Mantels (2) außerhalb des Mantels (2) durch Führungslöcher (5, 6) verläuft, insbesondere Papillotom, wobei der Mantel (2) bezüglich zweier rechtwinklig zueinander stehender Querschnittsrichtungen unterschiedliche Widerstandsmomente gegen Biegung aufweist, und das Zugelement (3) auf der Seite angeordnet ist, zu der hin der Mantel (2) das geringere Widerstandsmoment aufweist, dadurch gekennzeichnet, daß die Führungslöcher (5, 6) derart angeordnet sind, daß das Zugelement (3) im Krümmungsabschnitt (B) außerhalb (auf der einen oder anderen Seite) und schräg zu einer mittleren bzw. resultierenden Biegeebene (17, $E_1$) verläuft.

2. Sonde nach Anspruch 1, dadurch gekennzeichnet, daß der Mantel (2) einen länglichen, insbesondere rechteckigen oder ovalen Querschnitt aufweist.

3. Sonde nach Anspruch 2,
dadurch gekennzeichnet,
daß der Mantel durch ein Rohr gebildet ist, daß im Krümmungsabschnitt (B) flach gedrückt ist.

4. Sonde nach einem der Ansprüche 1 bis 3,
dadurch gekennzeichnet,
daß dem Mantel ein Biegeelement (8) zugeordnet ist, daß bezüglich zwei rechtwinklig zueinander stehenden Richtungen seines Querschnitts unterschiedliche Widerstandsmomente aufweist und so angeordnet ist, daß das Zugelement (3) auf der Seite angeordnet ist, zu der hin das Biegelement (8) das geringere Widerstandsmoment aufweist.

5. Sonde nach Anspruch 4,
dadurch gekennzeichnet,
daß das Biegeelement eine Blattfeder (8) ist.

6. Sonde nach Anspruch 4 oder 5,
dadurch gekennzeichnet,
daß das Biegeelement (8) im Mantel (2) angeordnet ist.

7. Sonde nach einem der Ansprüche 4 bis 6,
dadurch gekennzeichnet,
daß das Zugelement (3) am inneren Ende des Biegeelementes (8) befestigt ist.

8. Sonde nach einem der Ansprüche 4 bis 7,
dadurch gekennzeichnet,
daß das äußere Ende des Biegeelements (8) sich mit einem Verlängerungsabschnitt (21) über den Biegeabschnitt (B) hinaus erstreckt.

9. Sonde nach Anspruch 8,
dadurch gekennzeichnet,
daß der Verlängerungsabschnitt quer zur Ebene ($E_2$) der Blattfeder (8) gewellt oder zickzack-förmig geformt ist.

10. Sonden-Sortiment mit Sonden nach wenigstens einem der Ansprüche 1 bis 9, dadurch gekennzeichnet, daß es wenigstens eine Sonde (11) mit in der mittleren bzw. resultierenden Biegeebene (17, $E_1$) verlaufendem Zugelement (3, 18) und wenigstens eine Sonde (1) aufweist, bei der das Zugelement (3, 18) auf der einen oder der anderen Seite der mittleren bzw. resultierenden Biegeebene (17, $E_1$) verläuft.

11. Sonden-Sortiment nach Anspruch 10,
dadurch gekennzeichnet,
daß die Sonden (1) unterschiedliche Schnittwinkel ($w_1$ bis $w_3$) aufweisen.

## Claims

1. A probe (1) for introduction into the human or animal body, in particular a papillotome, having a sheath (2) of flexible material and a traction element (3) that extends longitudinally in the sheath and, for the purpose of effecting curvature by exerting traction at its outer end, is fastened to the sheath (2) and runs through guide holes (5, 6) outside the sheath (2) in the region of the curvature (B) of the sheath (2), wherein the sheath (2) has different moments of resistance to bending in two mutually perpendicular directions across its cross-section, and that the traction element (3) is arranged on the side towards which the sheath (2) has the smaller moment of resistance, characterised in that the guide holes (5, 6) are arranged so that the traction element (3) runs outside of (on one or the other side) and obliquely to a middle or resulting bending plane (17, $E_1$) in the region of curvature (B).

2. A probe according to claim 1, characterised in that the sheath (2) has an elongated, in particular rectangular or oval, cross-section.

3. A probe according to claim 2, characterised in that the sheath comprises a tube that is flattened in the region of curvature (B).

4. A probe according to any one of claims 1 to 3, characterised in that the sheath has associated with it a bending element (8) that has different resistance moments in respect of two mutually perpendicular directions across its cross-section and is so arranged that the traction element (3) is arranged on the side towards which the bending element (8) exhibits the smaller resistance moment.

5. A probe according to claim 4, characterised in that the bending element is a leaf spring (8).

6. A probe according to claim 4 or claim 5, characterised in that the bending element (8) is arranged in the sheath (2).

7. A probe according to any one of claims 4 to 6, characterised in that the traction element (3) is fastened to the inner end of the bending element (8).

8. A probe according to any one of claims 4 to 7, characterised in that an extension (21) on the outer end of the bending element (8) extends beyond the bending section (B).

9. A probe according to claim 8, characterised in that the extension has a corrugated or zig-zag shape transverse to the plane ($E_2$) of the leaf spring (8).

10. A set of probes according to at least one of claims 1 to 9, characterised in that it includes at least one probe (11) having a traction element (3, 18) extending in the middle or resulting bending plane (17, $E_1$) and at least one probe (1) of which the traction element (3, 18) runs on one or the other side of the middle or resulting bending plane (17, $E_1$).

**11.** A set of probes according to claim 10, characterised in that the probes (1) have different cutting angles ($w_1$ to $w_3$).

**Revendications**

**1.** Sonde (1) à introduire dans le corps humain ou d'un animal, avec une gaine (2) en matière flexible, et avec un élément de traction (3) s'étendant longitudinalement dans la gaine et qui est fixé à la gaine (2) dans le but de provoquer une courbure en exerçant une traction sur son extrémité extérieure, et qui s'étend hors de la gaine (2) dans la région de courbure (B) de la gaine (2) en passant par des trous de guidage (5, 6), en particulier un papillotome, tandis que la gaine (2) présente par rapport à deux directions transversales perpendiculaires entre elles des moments différents de résistance à la flexion, et que l'élément de traction (3) est disposé sur le côté vers lequel la gaine (2) présente le moindre moment de résistance,

**caractérisée** en ce que les trous de guidage (5, 6) sont disposés de telle sorte que l'élément de traction (3) s'étende dans la région de courbure (B) à l'extérieur (sur l'un ou l'autre côté) et obliquement par rapport à un plan de flexion (17, $E_1$) médian ou résultant.

**2.** Sonde selon la revendication 1, caractérisée en ce que la gaine (2) présente une section transversale allongée, en particulier rectangulaire ou ovale.

**3.** Sonde selon la revendication 2, caractérisée en ce que la gaine est constituée d'un tube qui est comprimé à plat dans la région de courbure (B).

**4.** Sonde selon une des revendications 1 à 3, caractérisée en ce qu'un élément flexible (8) est associé à la gaine, cet élément présentant, par rapport à deux directions perpendiculaires entre elles de sa section transversale, des moments de résistance différents et étant disposé de telle sorte que l'élément de traction (3) est disposé sur le côté par rapport auquel l'élément flexible (8) présente le moindre moment de résistance.

**5.** Sonde selon la revendication 4, caractérisée en ce que l'élément flexible est une lame de ressort (8).

**6.** Sonde selon la revendication 4 ou 5, caractérisée en ce que l'élément flexible (8) est disposé dans la gaine (2).

**7.** Sonde selon une des revendications 4 à 6, caractérisée en ce que l'élément de traction (3) est fixé à l'extrémité intérieure de l'élément flexible (8).

**8.** Sonde selon une des revendications 4 à 7, caractérisée en ce que l'extrémité extérieure de l'élément flexible (8) s'étend au delà de la région de courbure (B) par une section de prolongement (21).

**9.** Sonde selon la revendication 8, caractérisée en ce que la section de prolongement présente des ondulations ou une formation en zigzag, transversalement au plan ($E_2$) de la lame de ressort (8).

**10.** Assortiment de sondes avec des sondes selon au moins une des revendications 1 à 9, caractérisé en ce qu'il présente au moins une sonde (11) avec un élément de traction (3, 18) s'étendant dans le plan médian ou le plan résultant de flexion (17, $E_1$), et au moins une sonde (1) dans laquelle l'élément de traction (3, 18) s'étend sur l'un ou l'autre côté du plan médian ou du plan résultant de flexion (17, $E_1$).

**11.** Assortiment de sondes selon la revendication 10, caractérisé en ce que les sondes (1) présentent des angles de coupe ($W_1$ à $W_3$) différents.

FIG.1

FIG.2

3,12

5

2

9

18

8

14

a,B

III

III

6

4

11

17,E₁

E₃

E₃

2

18

8,E₂

W₁

FIG. 4

17,E₁

18

2

8,E₂

W₁

FIG. 3

17

3,18

15

14

E₁,E₃

2

E₂,8

FIG.3

W₃

8,E₂

2

17,E₁

FIG. 5

17,E₁

2

FIG. 6

FIG. 7

FIG. 8

FIG. 9

FIG. 10